# EUROPEAN PATENT SPECIFICATION

(11) **EP 2 654 927 B1**
(45) Date of publication and mention of the grant of the patent: **13.12.2017**
(21) Application number: 10812813.3
(22) Date of filing: 22.12.2010
(51) Int. Cl.: B01D 53/86, B01D 53/88, A61L 9/20

(54) **DEVICE FOR REDUCING POLLUTANTS IN A GAS MIXTURE**
VORRICHTUNG ZUR SCHADSTOFFREDUKTION IN EINEM GASGEMISCH
DISPOSITIF POUR RÉDUIRE LES POLLUANTS DANS UN MÉLANGE GAZEUX

(43) Date of publication of application: 30.10.2013
(73) Proprietor: Bitossi, Marco, 50056 Montelupo Fiorentino (Fl) (IT)
(72) Inventor: BALDI, Giovanni, I-50025 Montespertoli - Fl (IT); CIONI, Andrea, I-50053 Empoli - Fl (IT); DAMI, Valentina, I-51036 Larciano - PT (IT)
(74) Representative: Bellomia, Paolo
(86) International application number: PCT/IT2010/000509
(87) International publication number: WO 2012/085950

(56) References cited:
- EP-A1- 1 281 431
- WO-A1-2011/010627
- JP-A- 2004 305 854
- JP-A- 2005 205 094
- JP-A- 2007 007 588
- US-A1- 2004 007 453
- US-A1- 2010 260 644
- US-B1- 6 797 127

## Description

### Technical Field of the Invention

The present invention refers to a device for reducing pollutants in a gas mixture.

The protection of people's health, both inside and outside buildings, is presently a serious problem for the solution of which many firms and companies invest heavily also to comply with European directives intended to improve and sanitize work and dwell environments.

The majority of people spend most of their time within closed environments.

Several studies have made evident that the level of pollutants in a closed place-may be equal to and even higher than that present outside: actually, to the usual polluting substances coming from the outside, such as nitrogen oxides, sulphur oxides, carbon oxides and particulates, other polluting agents can be added, so-called volatile organic substances (VOS), for example formaldehyde and benzene, which are generated by cleaning products, gas hot plates, smoke and furnitures.

As far as the polluting substances present outside the buildings are concerned, particularly dangerous are the nitrogen oxides (NOx) produced by combustion processes, for example.

High concentrations of VOS and NOx can cause such health problems as allergic reactions or more serious symptoms.

For this reason, there is a great interest in searching a way for improving the quality of closed environments by treating internal and external surfaces of buildings, for example, with photocatalytic substances able to drop the level of polluting substances present in the air.

### State of the Art

Presently, an air cleaning system is based on the use of photocatalytic mortars or cement paints, that is, mortars containing chemical compounds capable of easily reacting, in the presence of UV radiations, with some pollutants and allowing the latter to be removed by direct absorption.

The exposition of said coatings to UV radiation brings about the formation of particles which act as catalysts for oxidoreduction and reduction reactions, thereby transforming the pollutants into chemical species having reduced environmental impact.

A photocatalytic substance usually employed for this purpose is titanium dioxide which, in the presence of UV radiation, is able to photocatalytically destroy the polluting substances by either degrading them down to water and CO₂, or mineralizing them to nontoxic substances. Portable systems also exist comprising photocatalytic filters, made from textile or paper material coated with titanium oxide, which are of more flexible and prompt use with respect to the above mentioned photocatalytic mortars or cement paints. **Example of this kind of devices can be found, for example, in the documents** JP2007007588 **and** US2004007453. Unfortunately, the portable systems which use such filters imply significant losses of load and are subjected to a noticeable wear, inasmuch as the titanium dioxide corrodes both the fabric and paper as time goes by. In addition, some difficulty has resulted in illuminating such filters uniformly because of their porous nature.

### Detailed Description

In this context, the specific technical object of the present invention is to propose a device for reducing pollutants in a gas mixture, able to overcome the above indicated drawbacks. In particular, the object of the present invention is to provide a device for reducing pollutants in a gas mixture, said device being able to resist wear and not implying significant load losses. A further object of the present invention is to
provide a device for reducing pollutants in a gas mixture, easily to use and operatively flexible in closed and open environments.

The indicated technical task and the specified objects are substantially achieved by a device for reducing pollutants in a gas mixture which comprises the technical characteristics set forth in one or more of the appended claims.

Further characteristics and advantages of the present invention will appear more clearly from the indicative, and thus non-limiting, preferred, but non-exclusive, embodiment of a device for reducing pollutants in a gas mixture, as illustrated in the accompanying drawings, wherein:

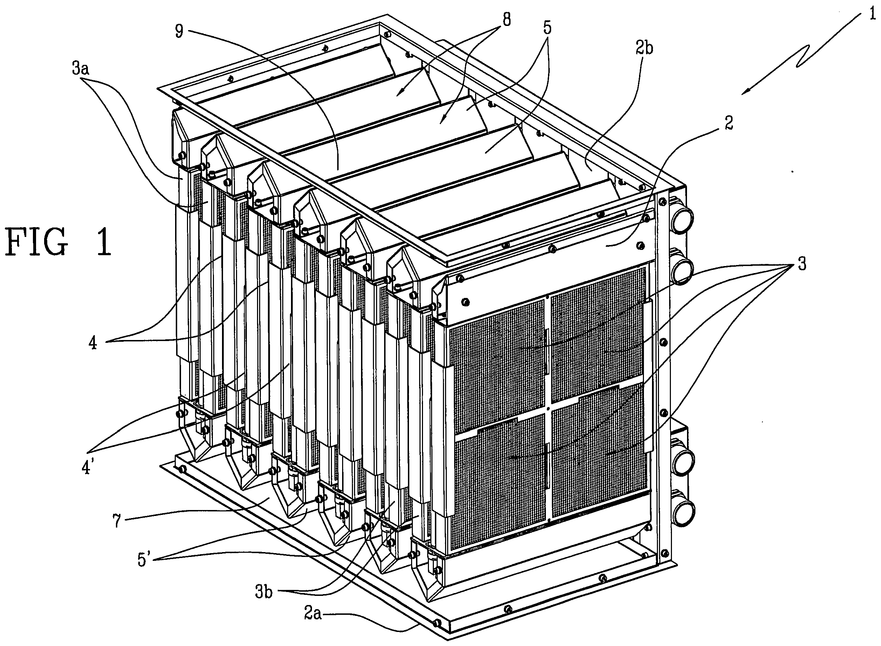

- Fig. 1 is a schematic view of the device for reducing pollutants in a gas mixture, according to the present invention; and
- Fig. 2 is a schematic front view of the device in Fig. 1.

With reference to the attached figures, numeral 1 indicates on the whole a device for reducing pollutants in a gas mixture.

Such a device 1 comprises a housing body 2 having an inlet 2a and an outlet 2b, with at least one pair of filters 3 thereinside.

Since these are photocatalytic filters 3, inside the device 1 a first light source 4 is also provided of fluorescent or LED type and advantageously interposed between the filters 3 to uniformly illuminate them.

Each filter 3, advantageously panel-shaped, is a ceramic honeycomb shaped support coated with nano-particles of titanium dioxide, for example. In one embodiment, the two filters 3 face each other and, advantageously, are disposed in parallel relationship at a preset distance from each other.

In a second embodiment, the two filters 3 are disposed inclined with respect to the direction of flow F.

Moreover, advantageously, the device 1 comprises a drive means for moving said filters 3 so as to change the angle between a pair of same filters 3 according to the characteristics of the air flow to be cleaned up.

In correspondence of a first respective end 3a, the two filters 3 are connected to each other by a first closing element 5 which delimitates the opening between the two filters 3. At a second end 3b, opposite to the first 3a, the two filters 3 are not connected to each other and, accordingly, define an open section. A space 6 is thereby provided for the passage of the gas mixture to be cleaned, said space 6 being defined by the walls of the two filters 3 and the first closing element 5. The space 6 is then closed laterally by the internal walls of the housing body 2.

The open section defines, in correspondence of the second ends 3b of filters 3, the entry section 7 of space 6 facing the inlet 2a of housing body 2 and from which the gas mixture to be cleaned is introduced.

The first light source 4 is held inside such space 6, preferably projecting from the first closing element 5 with which is electrically connected. In this way the light source 4 illuminates homogeneously the mutually facing inner surfaces 3c of the two filters 3.

The whole of the two facing-each-other filters 3, first closing element 5 and first light source 4 interposed between the two filters 3, makes up a filtering module 8 disposed in such a way that the entry section of space 6 is facing and close to the inlet 2a of the housing body 2.

The device 1 advantageously comprises a plurality of filtering modules 8 disposed side-by-side at a preset distance so that the outer surfaces of two filters 3, belonging to two distinct adjacent filtering module 8, will face to each other.

Each filtering module 8 is connected to the adjacent module, in correspondence of the second end 3b of the respective filters 3 side-by-side disposed, through a second closing element 5' which delimitates the opening between the filters 3 of the two side-by-side disposed modules 8.

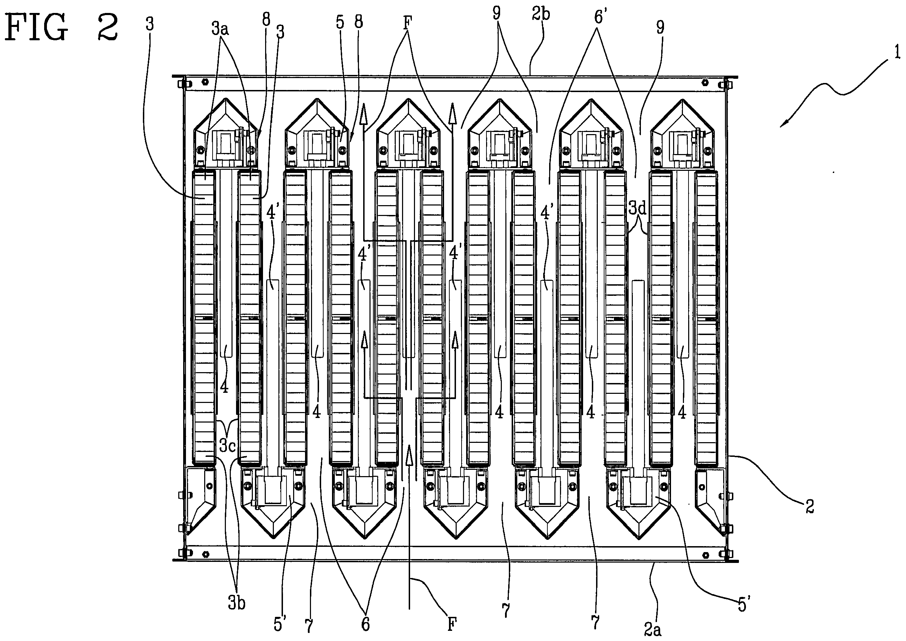

Also in this case a further space 6' is created to allow the gas mixture already cleaned to pass through, said space being defined by the outer surfaces 3d of two adjacent filtering modules 8, and the second closing element 5'. Also in this case, the space 6' is laterally delimitated by filters 3. Interposed between each module 8 is a second light source 4' to ensure a homogenous distribution of light over the whole outer surface 3d of filter 3. As can be seen in Fig. 2, each second light source 4' is connected to the second closing element 5' inside which the electrical connection can be advantageously prearranged. The space 6', therefore, exhibits an open exit section 9 facing the outlet 2b of the housing body 2 and close to same outlet 2b.

With reference to Fig. 2, the arrows F indicate the way the gas mixture to be cleaned enters the device 2 through the inlet 2a, goes into the space 6 from entry section 7 and through the honeycomb filters 3, whose pollutant-reducing capacity is activated by the light from sources 4, 4' and, finally, comes out of the exit section 9 of space 6' between two adjacent filters 8 to leave already cleaned the device 1 through the outlet 2b.

In other words, the device 1 exhibits a plurality of filters 3 disposed in succession with alternating light sources 4, 4'.

The union between two filters 3 is made by connecting in alternate sequence - via a closing element 5, 5' to which a respective light source 4, 4' is connected - the first ends 3a and second ends 3b, so that one filter 3, in intermediate position along the sequence, be connected to the preceding filter 3 through the first end 3a and to the following filter 3 through the second end 3b.

Advantageously, the light sources 4, 4' used inside the device exhibit a wavelength between 300 and 400 nm, preferably of 351 nm. Such value has been experimentally identified by means of suitable apparatuses able to evaluate the degree of reduction of certain polluting substances such as nitrogen oxides (NOx) and volatile organic substances (VOC).

Such value makes it possible to reduce the polluting substances by 90-95%.

Kinematic models have revealed that with a device 1 having a filtering surface of about 23 m² it is possible to reduce by 95% the concentration of pollutants in a gas mixture having a volume of 24000 m³/day and initial concentration of 50 ppb.

The filters' support material is made of ceramic, such as pyroceram, alumina, cordierite, mullite and other ceramic materials.

From what has been set forth above it appears evident that the invention achieves the proposed objects and important advantages as well.

In fact, the described device is able to reduce a significant concentration of pollutants while avoiding the drawbacks of the prior art.

In fact, the filters being used withstand the titanium dioxide coating and do not undergo any deterioration. The device does not exhibit load losses and, since it is portable, can be advantageously used both indoors and outdoors.

The path followed by the gas mixture within the device ensures an optimal degree of reduction of polluting substances.

The excellent results being obtained are due in part also to the particular wave length employed.

## Claims

1. Device for reducing pollutants in a gas mixture, comprising a housing body (2) having an inlet (2a) and an outlet (2b), at least one pair of filters (3) disposed inside the housing body (2), a first light source (4) interposed between said filters (3), wherein said filters (3) are connected to each other in correspondence of one respective end (3a, 3b) by a first closing element (5); said pair of filters (3) and said first closing element (5) defining a space (6) for the passage of said gas mixture which has only one open section (7) facing the inlet (2a) of said housing body (2); **characterized in that** each filter (3) is a honeycomb shaped ceramic support coated with photocatalytic nanoparticles.

2. Device according to the preceding claim, **characterized in that** said first light source (4) has a wave length between 300 and 400 nm.

3. Device according to claim 1 or 2, **characterized in that** said first light source (4) has a wave length of 351 nm.

4. Device according to any of the previous claims, **characterized in that** said first light source (4) is held inside said space (6).

5. Device according to any of the previous claims, **characterized in that** said first light source (4) is connected to said closing element (5) holding electrical connections thereinside.

6. Device according to any of the previous claims, **characterized in that** it comprises a filtering module (8) made up of said two filters (3) facing each other in parallel relationship, of said first closing element (5) and of said first light source (4); said filtering module (8) being disposed inside the housing body (2), with said first closing element (5) being in correspondence of the outlet (2b) of said housing body (2).

7. Device according to claim 6, **characterized in that** it comprises, inside said housing body (2), a plurality of filtering modules (8) disposed side-by-side at a preset distance from each other, with the respective filters (3) being parallel to each other.

8. Device according to claim 7, **characterized in that** the filtering modules (8) are connected to each other by a second closing element (5') located in correspondence of the second ends (2b) of the respective side-by-side disposed filters (3).

9. Device according to claim 8, **characterized in that** it comprises a second light source (4') located between two different side-by-side disposed filters (3) and connected to the second closing element (5').

10. Device according to claim 9, **characterized in that** said second light source (4') has a wave length between 300 and 400 nm.

11. Device according to claim 9 or 10, **characterized in that** said second light source (4') has a wave length of 351 nm.

## Patentansprüche

1. Vorrichtung zum Reduzieren von Schadstoffen in einem Gasgemisch, umfassend einen Gehäusekörper (2) mit einem Einlass (2a) und einem Auslass (2b), mindestens einem innerhalb des Gehäusekörpers (2) angeordneten Filterpaar (3), eine zwischen den Filtern (3) angeordnete Lichtquelle (4), wobei die Filter (3) an einem jeweiligen Ende (3a, 3b) durch ein erstes Schliesselement (5) miteinander verbunden sind; wobei das Filterpaar (3) und das erste Schliesselement (5) einen Raum (6) für den Durchgang des Gasgemischs definieren, der nur einen dem Einlass (2a) des Gehäusekörpers (2) zugewandten offenen Abschnitt (7) aufweist; **dadurch gekennzeichnet, dass** jeder Filter (3) ein wabenförmiger keramischer Träger ist, der mit photokatalytischen Nanopartikeln beschichtet ist.

2. Vorrichtung nach dem vorhergehenden Anspruch, **dadurch gekennzeichnet, dass** die erste Lichtquelle (4) eine Wellenlänge zwischen 300 und 400 nm aufweist.

3. Vorrichtung nach Anspruch 1 oder 2, **dadurch gekennzeichnet, dass** die erste Lichtquelle (4) eine Wellenlänge von
351 nm aufweist.

4. Vorrichtung nach einem der vorhergehenden Ansprüche, **dadurch gekennzeichnet, dass** die erste Lichtquelle (4) innerhalb des Raums (6) gehalten wird.

5. Vorrichtung nach einem der vorangehenden Ansprüche, **dadurch gekennzeichnet, dass** die erste Lichtquelle (4) mit dem Schliesselement (5) verbunden ist, das die elektrischen Anschlüsse darin hält.

6. Vorrichtung nach einem der vorhergehenden Ansprüche, **dadurch gekennzeichnet, dass** sie ein Filtermodul (8) aus den zwei Filtern (3), die in paralleler Beziehung einander zugewandt sind, dem ersten Schliesselement (5) und der ersten Lichtquelle (4) zusammengesetzt ist; wobei das Filtermodul (8) innerhalb des Gehäusekörpers (2) angeordnet ist, wobei das erste Schliesselement (5) am Auslass (2b) des Gehäusekörpers (2) ist.

7. Vorrichtung nach Anspruch 6, **dadurch gekennzeichnet, dass** sie innerhalb des Gehäusekörpers (2) eine Vielzahl von Filtermodulen (8) umfasst, die in einem voreingestellten Abstand nebeneinander angeordnet sind, wobei die jeweiligen Filter (3) parallel zueinander sind.

8. Vorrichtung nach Anspruch 7, **dadurch gekennzeichnet, dass** die Filtermodule (8) durch ein zweites Schliesselement (5') miteinander verbunden sind, das an den zweiten Enden (2b) der jeweiligen nebeneinander angeordneten Filter (3).

9. Vorrichtung nach Anspruch 8, **dadurch gekennzeichnet, dass** sie eine zweite Lichtquelle (4') umfasst, die zwischen zwei verschiedenen nebeneinander angeordneten Filtern (3) angeordnet und mit dem zweiten Schliesselement (5') verbunden ist.

10. Vorrichtung nach Anspruch 9, **dadurch gekennzeichnet, dass** die zweite Lichtquelle (4') eine Wellenlänge zwischen 300 und 400 nm aufweist.

11. Vorrichtung nach Anspruch 9 oder 10, **dadurch gekennzeichnet, dass** die zweite Lichtquelle (4') eine Wellenlänge von 351 nm aufweist.

## Revendications

1. Dispositif servant à réduire les polluants dans un mélange gazeux, comprenant un corps de logement (2) comportant une entrée (2a) et une sortie (2b), au moins une paire de filtres (3) disposés à l'intérieur du corps de logement (2), une première source lumineuse (4) interposée entre lesdits filtres (3), dans lequel lesdits filtres (3) sont reliés l'un à l'autre, en correspondance d'une extrémité (3a, 3b) respective, par un premier élément de fermeture (5) ; ladite paire de filtres (3) et ledit premier élément de fermeture (5) définissant un espace (6) pour le passage dudit mélange gazeux ne comportant qu'une seule section d'ouverture (7) faisant face à l'entrée (2a) dudit corps de logement (2) ; **caractérisé en ce que** chaque filtre (3) est un support céramique en forme de nid d'abeilles revêtu de nanoparticules photocatalytiques.

2. Dispositif selon la revendication précédente, **caractérisé en ce que** ladite première source lumineuse (4) possède une longueur d'onde comprise entre 300 et 400 nm.

3. Dispositif selon la revendication 1 ou 2, **caractérisé en ce que** ladite première source lumineuse (4) possède une longueur d'onde de
351 nm.

4. Dispositif selon l'une quelconque des revendications précédentes, **caractérisé en ce que** ladite première source lumineuse (4) est maintenue à l'intérieur dudit espace (6).

5. Dispositif selon l'une quelconque des revendications précédentes, **caractérisé en ce que** ladite première source lumineuse (4) est reliée au dit élément de fermeture (5) maintenant des connexions électriques en son sein.

6. Dispositif selon l'une quelconque des revendications précédentes, **caractérisé en ce qu'**il comprend un module de filtrage (8) constitué desdits deux filtres (3) se faisant face dans une relation en parallèle, dudit premier élément de fermeture (5) et de ladite première source lumineuse (4) ; ledit module de filtrage (8) étant disposé à l'intérieur du corps de logement (2) avec ledit premier élément de fermeture (5) étant en correspondance de la sortie (2b) dudit corps de logement (2).

7. Dispositif selon la revendication 6, **caractérisé en ce qu'**il comprend, à l'intérieur dudit corps de logement (2), une pluralité de modules de filtrage (8) disposés côte à côte à une distance préétablie les uns des autres avec les filtres (3) respectifs étant parallèles les uns aux autres.

8. Dispositif selon la revendication 7, **caractérisé en ce que** les modules de filtrage (8) sont reliés les uns aux autres par un second élément de fermeture (5') situé en correspondance des secondes extrémités (2b) des filtres (3) respectifs disposés côte à côte.

9. Dispositif selon la revendication 8, **caractérisé en ce qu'**il comprend une seconde source lumineuse (4') située entre deux filtres (3) différents disposés côte à côte et reliée au second élément de fermeture (5').

10. Dispositif selon la revendication 9, **caractérisé en ce que** ladite seconde source lumineuse (4') possède une longueur d'onde comprise entre 300 et 400 nm.

11. Dispositif selon les revendications 9 ou 10, **caractérisé en ce que** ladite seconde source lumineuse (4') possède une longueur d'onde de 351 nm.
